# EUROPÄISCHE PATENTSCHRIFT

(11) **EP 1 281 079 B1**
(45) Veröffentlichungstag und Bekanntmachung des Hinweises auf die Patenterteilung: **22.11.2006**
(21) Anmeldenummer: 01949322.0
(22) Anmeldetag: 08.05.2001
(51) Int. Cl.: G01N 33/00, G01N 33/28

(54) **VERFAHREN UND VORRICHTUNG ZUR ÜBERWACHUNG VON GASEN**
METHOD AND DEVICE FOR THE MONITORING OF GASES
PROCEDE ET DISPOSITIF DE CONTROLE DE GAZ

(30) Priorität: 10.05.2000 DE 10022714
(43) Veröffentlichungstag der Anmeldung: 05.02.2003
(73) Patentinhaber: Bräsel, Eckhard, 17493 Greifswald (DE)
(72) Erfinder: BRÄSEL, Eckhard, 17493 Greifswald (DE); SASUM, Ute, 17491 Greifswald (DE)
(74) Vertreter: Grünecker, Kinkeldey, Stockmair & Schwanhäusser Anwaltssozietät
(86) Internationale Anmeldenummer: PCT/EP2001/005223
(87) Internationale Veröffentlichungsnummer: WO 2001/086284

(56) Entgegenhaltungen:
- EP-A- 0 525 933
- EP-A- 0 534 331
- DD-A- 135 158
- DD-A- 254 827
- DE-A- 4 136 639
- DE-C- 19 833 601
- US-A- 3 942 792

## Beschreibung

Die Erfindung betrifft Verfahren zur Überwachung von Gasen in flüssigkeitsgefüllten Hochspannungsanlagen und eine entsprechende Vorrichtung.

Hochspannungsanlagen, im speziellen Großtransformatoren, werden mit Flüssigkeit, meistens Öl, gekühlt. Das Öl sollte überwacht werden, um Fehlfunktionen detektieren zu können. Durch Analyse der auftretenden Gase ist es möglich, herauszufinden, welches Gas im Öl vorhanden ist und mit welcher Konzentration. Aus der Kenntnis des im Öl befindlichen Gases können dann Rückschlüsse auf den Grund für die Bildung des Gases gezogen werden. So ist es z.B. wohlbekannt, dass das Auftreten von Wasserstoffgas im Öl eines Transformators auf das Auftreten von elektrischen Entladungen innerhalb des Transformators hinweist.

Andererseits ist freies, ungelöstes Gas in der Transformatorflüssigkeit zum einen eine Gefahr für die Durchschlagsfestigkeit des Isolationssystems und zum anderen ein Informationsträger für die Ursache seiner Entstehung. Ist solches freies Gas vorhanden, sollte eine möglichst schnelle Gasanalyse möglich sein.

Zur optimalen Überwachung von flüssigkeitsgefüllten Hochspannungsanlagen ist daher Kenntnis über den Gesamtgasgehalt des Öles, der Zusammensetzung des gelösten Gases, die Feststellung ungelöster Gase und/oder die Zusammensetzung der ungelösten Gase wünschenswert.

In EP 0534331 A1 ist eine Vorrichtung geschildert, mit deren Hilfe im Öl vorhandene Gase festgestellt werden sollen, indem eine Druckmessung in Kombination mit Wärmeleitfähigkeitsmessungen des vorhandenen Gases in einem isochoren Gleichgewichtsgasraum durchgeführt wird. Öl wird über eine Glasfritte zugeführt, so dass ein Staudruck geschaffen wird. Bei gasgesättigten bzw. gasübersättigten Ölen kann das Verfahren daher nicht angewendet werden.

Ein Verfahren zur Detektion ungelöster Gase unter Einsatz einer Membran ist in DE 198 33 601 C1 beschrieben.

Aufgabe der vorliegenden Erfindung ist es, ein verbessertes Verfahren zur Überwachung von gelösten Gasen in flüssigkeitsgefüllten Hochspannungsanlagen anzugeben, mit dessen Hilfe auf einfache Weise eine Gasanalyse der im Öl gelösten Gase möglich ist bzw. ein Verfahren anzugeben, mit dem eine Analyse der im Öl gelösten und ungelösten Gase möglich' ist Weiterhin ist es Aufgabe der Erfindung, eine Vorrichtung zur Durchführung der erfindungsgemäßen Verfahren anzugeben.

Diese Aufgaben werden mit Hilfe eines Verfahrens mit den Merkmalen eines der Ansprüche 1, 6 oder 7 bzw. ein Verfahren mit den Merkmalen des Anspruchs 8 und einer Vorrichtung mit den Merkmalen des Anspruchs 14 gelöst. Vorteilhafte Ausgestaltungen sind Gegenstand der jeweiligen Unteransprüche.

Bei dem erfindungsgemäßen Verfahren zur Überwachung von gelösten Gasen in flüssigkeitsgefüllten Hochspannungsanlagen steht ein Separiergefäß mit dem Flüssigkeitstank der Hochspannungsanlage in Verbindung. In dem Separiergefäß herrscht im Normalbetrieb ein Gleichgewichtszustand zwischen der Flüssigkeit und dem Gaszustand. Mit einer Pumpe wird der Flüssigkeitsspiegel auf einer vorbestimmten Höhe gehalten, wobei die Pumpe dabei im Separiergefäß unterhalb des Flüssigkeitsspiegels angreift.

Durch kurzzeitiges Unterbrechen des Pumpvorganges oder Verringerung der Pumpleistung erhöht sich der Flüssigkeitsspiegel und der Gleichgewichtszustand wird kurzzeitig verlassen. Oberhalb des Flüssigkeitsspiegels im Gasraum des Separiergefäßes erhöht sich der Druck leicht bis oberhalb des Umgebungsdrucks, so dass die Entnahme einer Gasprobe aus dem Gasraum zur Analyse durch eine oberhalb des Flüssigkeitsspiegels des Separierraums angeordnete Probeentnahmestelle möglich ist

Dabei ist mit dem Ausdruck Entnahme sowohl die Entnahme einer Gasprobe zur externen Analyse als auch die Entnahme von Gas gemeint, das direkt einem Gasanalysesensor zugeführt wird, so dass eine direkte "Online"-Analyse möglich ist.

Mit der Erfindung ist es auf einfache Weise möglich, die in dem Öl gelösten Gase zu überwachen. Die Messung ist einfach, schnell und leicht durchführbar, so dass sie häufig durchgeführt werden kann. Andererseits treten messbare Änderungen im Kesselöl langzeitiger auf, normalerweise in der Größenordnung von einem Tag. Insofern ist eine kontinuierliche Überwachung gewährleistet. Die kurzzeitigen Störungen des Gleichgewichtsgaszustandes bei den Gasprobenentnahmen sind auf Grund dieser unterschiedlichen Zeitskalen ebenfalls unproblematisch. Die entnommene Gasprobe ermöglicht dann auf einfache Weise eine Analyse des ölgelösten Gases z.B. zu einer Überwachung bzw. einer Störungsanalyse.

Die Gasprobe wird oberhalb des Flüssigkeitsspiegels entnommen. Dabei kann das Gas z.B. manuell durch ein Septum bzw. einen Sperrhahn oder mit Hilfe einer automatischen Gasentnahmepatrone, die ab einem gewissen Druck anspricht, entnommen werden. Während eine Gasentnahmepatrone eine automatische und einfache Entnahme gewährleistet, kann eine manuelle Entnahme ohne zusätzliche Geräte flexibel durchgeführt werden. Mit der entnommenen Probe ist eine Vollanalyse des im Öl gelösten Gases möglich. Zusätzlich dazu kann mit messgasverbrauchsfreien Sensoren, z.B. entsprechend modifizieren Wärmeleitungssensoren in Kombination mit selektiven IR-Sensoren, die Zusammensetzung der gelösten Gase bestimmt werden. Weiterhin kann mit einem entsprechend am Separiergefäß oberhalb des Flüssigkeitsspiegels angeordneten Druckmessgerät der Gassättigungsgrad bestimmt werden. Derartige Sensoren gewährleisten weitere Sicherheit bei der Analyse des im Öl gelösten Gases und können kontinuierlich im Einsatz sein.

Schließlich kann mit Hilfe einer Spülgasleitung, die über ein Ventil mit dem Gasraum des Separiergefäßes verbunden ist, Gas direkt zu einem Sensor geleitet werden.

Die erfindungsgemäßen Verfahren können periodisch durchgeführt werden. Dabei wird das Verfahren durchgeführt, im Anschluss daran auf die Wiedereinstellung des Gleichgewichts im Separiergefäß gewartet, um dann wiederum das Verfahren durchzuführen. Die Einstellung des Gleichgewichts nimmt in der Regel nur wenige Stunden, zum Beispiel zwei Stunden, in Anspruch, wohingegen die Änderungen im Kesselöl gewöhnlich langzeitiger sind. Dementsprechend ist eine kontinuierliche Überwachung des Kesselöls gewährleistet.

Mit Hilfe von messgasverbrauchsfreien Sensoren im Gasraum des Separiergefäßes oberhalb des Flüssigkeitsspiegels lässt sich die Zusammensetzung der im Öl gelösten Gase im Gleichgewichtszustand kontinuierlich bestimmen, auch ohne dass eine Probe entnommen werden müsste. Dabei kann auch ggf. durch kurzzeitiges Unterbrechen des Pumpvorganges oder Verringerung der Pumpleistung der Flüssigkeitsspiegel erhöht werden. So können ggf. definiertere und reproduzierbarere Druckverhältnisse erhalten werden, wenn die Messung durchgeführt wird.

Die erfindungsgemäßen Verfahren ermöglichen eine einfache Überwachung und Analyse der im Öl gelösten Gase. Treten zusätzlich ungelöste Gase auf, so spiegelt sich dies in den entnommenen Gasproben bzw. in den Signalen der Sensoren wieder. Eine Störung durch das Auftreten ungelöster Gase kann also schnell bemerkt werden und entsprechende Vorkehrungen zur Behebung getroffen werden.

Bei dem erfindungsgemäßen Verfahren zur Überwachung von gelösten und ungelösten Gasen an flüssigkeitsgefüllten Hochspannungsanlagen ist das Separiergefäß mit einer Entlüftung des Flüssigkeitstanks über eine weitere Zuführung verbunden, in der sich in einem Steigleitungsbereich ein Füllstandsensor befindet. Bei dessen Ansprechen im Falle des Auftretens von ungelösten Gasen wird das oben geschilderte erfindungsgemäße Verfahren eingesetzt um die gelösten Gase zu überwachen. Anschließend werden ungelöste Gase aus dem Flüssigkeitstank in das Separiergefäß eingeführt. Dabei wird die zeitliche Veränderung der Analysenwerte und daraus die Herkunft der ungelösten Gase bestimmt.

Aus zuvor ermittelten Erfahrungswerten lässt sich aus der zeitlichen Änderung der Analysenwerte der Grund für das Auftreten und die Zusammensetzung der ungelösten Gase bestimmen. Dazu können Gasproben entnommen werden. Eine einfache ununterbrochene Überwachung der Änderung ist möglich, wenn als Analysewerte die Signale von messgasverbrauchsfreien Sensoren eingesetzt werden, die sich im Separiergefäß oberhalb des Flüssigkeitsspiegels befinden.

Der erfindungsgemäße Ablauf ermöglicht die nahezu gleichzeitige Bestimmung der Zusammensetzung der gelösten Gase im Gleichgewicht und der ungelösten Gase des Öles. Durch Vergleich des ungelösten Gases mit den im Gleichgewichtszustand vorhandenen Werten für das gelöste Gas lässt sich direkt auf die Zusammensetzung des zusätzlich auftretenden Gases schließen. Dies lässt eine sichere Bestimmung der Herkunft des zusätzlich auftretenden ungelösten Gases zu. Durch die nahezu gleichzeitige Messung der gelösten Gase ist die Zusammensetzung des im Öl gelösten Gases im Gleichgewichtszustand ohne Störung der Sensorfunktionen durch ungelöste Gase möglich. Die nahezu gleichzeitige Messung gewährleistet zudem, dass die Messung der gelösten und ungelösten Gase unter den gleichen sonstigen Umweltbedingungen, z.B. der Temperatur, erfolgen. Eine sichere Analyse des auftretenden Gases ist somit gewährleistet.

Bei einer vorteilhaften Ausgestaltung des Verfahrens wird nach der Durchführung einer Gasentnahme des gelösten Gases aus dem Gleichgewichtszustand ungelöstes Gas in ein Gassammelgefäß gesammelt, das von der Zuführung in das Separiergefäß abzweigt. Nachdem das ungelöste Gas in das Separiergefäß eingelassen worden ist, kann über die Entlüftung dieses Gassammelgefäßes eine Gasprobe entnommen werden, die ausschließlich ungelöstes Gas enthält. Auf diese Weise ist zusätzlich eine Analyse des ungelösten Gases auf seine Zusammensetzung hin möglich.

Ungelöstes Gas, das in dem Flüssigkeitstank einer Hochspannungsanlage auftritt, kann z.B. an einem Gassammelstutzen des Flüssigkeitstanks entnommen werden. Vorteilhafterweise ist dabei der Gassammelstutzen in eine Steigleitung eingebaut, die den Flüssigkeitstank im oberen Bereich verlässt und zu dem Separiergefäß führt. Ungelöstes Gas wird dann an der Entlüftung des Gassammelstutzens entnommen. Im Normalzustand wird Öl dem Gassammelstutzen entnommen. So kann mit den beschriebenen Verfahren im Separiergefäß das im Öl gelöste Gas analysiert bzw. überwacht werden. Treten ungelöste Gase auf, so sammeln sich diese in dem Gasraum des Gassammelstutzens und können über dessen Entlüftung entnommen werden und dem Separiergefäß gemäß dem erfindungsgemäßen Verfahren zugeführt werden. Wird der Gassammelstutzen direkt oberhalb des Flüssigkeitstanks in die Steigleitung eingefügt, so ist eine besonders repräsentative Überwachung möglich, da sich die Gassammelstutzen in direkter Nähe des Öls im Flüssigkeitstank befindet

Ist die Hochspannungsanlage bereits mit einem Gassammelrelais zur Detektion von ungelösten Gasen, z.B. einem Buchholzrelais ausgestattet, kann direkt aus diesem Gassammelrelais das ungelöste Gas zur Analyse entnommen werden.

Wird ein solches bereits vorhandenes Gassammelrelais eingesetzt, so kann eine Nachrüstung durch einen zusätzlichen Gassammelstutzen vermieden werden.

Eine erfindungsgemäße Vorrichtung zur Überwachung von Gasen in flüssigkeitsgefüllten Hochspannungsanlagen umfasst ein Separiergefäß und eine Zuführung, die mit dem Separiergefäß und dem Flüssigkeitstank der Hochspannungsanlage in Verbindung steht, wobei die Zuführung in das Separiergefäß in einer Höhe mündet, die im Normalbetrieb oberhalb des Flüssigkeitsspiegels im Separiergefäß liegt. Weiterhin ist eine Pumpe vorgesehen, die an dem Separiergefäß an einer Stelle angreift, die unterhalb des Flüssigkeitsspiegels liegt. Das Separiergefäß umfasst zumindest ein Entnahmeventil oberhalb des Flüssigkeitsspiegels zur Entnahme von Gasproben und ein Höhenstandmesssystem zur Detektion des Höhenstandes.

In dem abgeschlossenen Tank des Separiergefäßes bildet sich ein Gleichgewichtszustand mit einem Flüssigkeitsspiegel, oberhalb dessen sich Gas der im Öl gelösten Gase und unterhalb des Flüssigkeitsspiegels entsprechende Flüssigkeit befindet. Mit Hilfe der Pumpe lässt sich der Höhenstand des Flüssigkeitsspiegels regulieren, wobei in der Regel im Gasraum oberhalb des Flüssigkeitsspiegels ein Unterdruck entsteht. Dabei kann die Pumpe bzw. das Ventil, das die Pumpe von dem Separiergefäß -abkoppeln kann, mit Hilfe eines entsprechenden Höhenstandsmeßsystems geregelt werden, wobei die Flüssigkeitsspiegelhöhe als Regelgröße dient. Wird eine vorbestimmte Höhenstandsmarke überschritten, so schaltet die Regelkreis die Pumpe in den Kreislauf, um den Flüssigkeitsspiegel wieder abzusenken. Sinkt der Flüssigkeitsspiegel andererseits unter eine vorbestimmte Höhenstandsmarke, so wird die Pumpe von dem Separiergefäß abgekoppelt, um ein Steigen des Flüssigkeitsspiegels zu erreichen.

Die erfindungsgemäße Vorrichtung erlaubt bei der Durchführung des erfindungsgemäßen Verfahrens zur Überwachung der gelösten Gase durch Entnahme von Gasproben an dem Entnahmeventil oberhalb des Flüssigkeitsspiegels die Analyse des im Gasraum befindlichen Gases. Mit der erfindungsgemäßen Vorrichtung lässt sich das erfindungsgemäße Verfahren zur Überwachung gelöster Gase dementsprechend auf leichte Weise durchführen.

Vorteilhafterweise umfasst das Separiergefäß oberhalb des Flüssigkeitsspiegels eine Zuführung mit einem Schaltventil, durch das Eichgas in das Separiergefäß eingelassen werden kann, um eventuell vorhandene Messsensoren zu eichen. So ist es möglich, dass durch Vergleich der tatsächlichen Messungen mit den Eichmessungen direkt auf die Gaszusammensetzung bzw. -konzentration geschlossen werden kann.

Nach einer Messung kann durch Einschalten der Pumpe der Flüssigkeitsspiegel wiederum abgesenkt werden, so dass sich wieder der Gleichgewichtszustand einstellt. Andererseits kann auch oberhalb des Flüssigkeitsspiegels durch eine entsprechende Spülgaskapillare Gas abgelassen werden, um dieses abgelassene Gas ggf. zu weiteren Analysen mit alternativen Detektoren einzusetzen.

Bei einer vorteilhaften Ausgestaltung, mit der auch ungelöste Gase detektiert werden können, ist eine weitere Zuführung in das Separiergefäß vorgesehen, die mit dem Flüssigkeitstank der Hochspannungsanlage in Verbindung steht. In der Zuführung befindet sich in einem Steigleitungsbereich ein Füllstandssensor und eine Ventileinrichtung zur alternativen Auswahl der ersten oder zweiten Zuführung. Der Füllstandssensor und die zweite Zuführung sind im Normalbetrieb von dem Separiergefäß abgekoppelt und der Füllstandssensor ist von Öl umgeben. Entstehen in dem Flüssigkeitstank ungelöste Gase, wird das Öl am Füllstandssensor verdrängt, so dass er anspricht. In diesem Fall wird das erfindungsgemäße Verfahren zur Überwachung der gelösten und ungelösten Gase durchgeführt.

Eine einfache Ausführungsform dieser erfindungsgemäßen Vorrichtung umfasst ein Dreiwegeventil als Ventileinrichtung. Ein Anschluss ist an die Zuführung zum Separiergefäß angeschlossen und zwei weitere Anschlüsse des Dreiwegeventils an die erste und zweite Zuführung aus dem Flüssigkeitstank.

Die erfindungsgemäße Vorrichtung kann mit der zweiten Zuführung an ein vorhandenes Gassammelrelais, z.B. ein Buchholzrelais, des Flüssigkeitstanks angeschlossen werden bzw. an die Entlüftung eines Gassammelstutzens, der sich in einer Steigleitung aus dem Flüssigkeitstank befindet. Die erste Zuführung ist mit dem Flüssigkeitstank direkt, z.B. an einer vorhandenen Ölentnahmestelle, bzw. über den Gassammelstutzen verbunden.

Dabei hat der Anschluss an einem Gassammelstutzen in der Steigleitung direkt oberhalb des Fiüssigkeitstanks den Vorteil, dass die Verhältnisse direkt im Flüssigkeitstank bestimmt werden, da der Gassammelstutzen in unmittelbarer Nähe des Flüssigkeitstanks angeordnet sein kann. Ein Anschluss an ein vorhandenes Gassammelrelais bietet den Vorteil, dass keine weitere Nachrüstung mit einem zusätzlichen Gassammelstutzen notwendig ist.

Bei einer weiteren vorteilhaften Ausgestaltung ist zumindest an der zweiten Zuführung ein separates Gassammelgefäß angeschlossen, das im oberen Bereich einen Anschluss zur Probenentnahme umfasst. Beim Auftreten von ungelösten Gasen kann dieses Gassammelgefäß in den Kreislauf geschaltet werden, so dass eine unabhängige Gasprobeentnahme und Analyse der Zusammensetzung des ungelösten Gases möglich ist.

Mit Bezug zu der anliegenden Figur wird im Folgenden eine bevorzugte Ausführungsform der Vorrichtung und des Verfahrens geschildert Dabei zeigt
- Fig. 1: den schematischen Aufbau einer Ausführungsform der erfindungsgemäßen Vorrichtung,
- Fig. 2: schematisch eine mögliche Anschlussvariante der erfindungsgemäßen Vorrichtung, und
- Fig. 3: eine weitere Anschlussvariante der erfindungsgemäßen Vorrichtung.

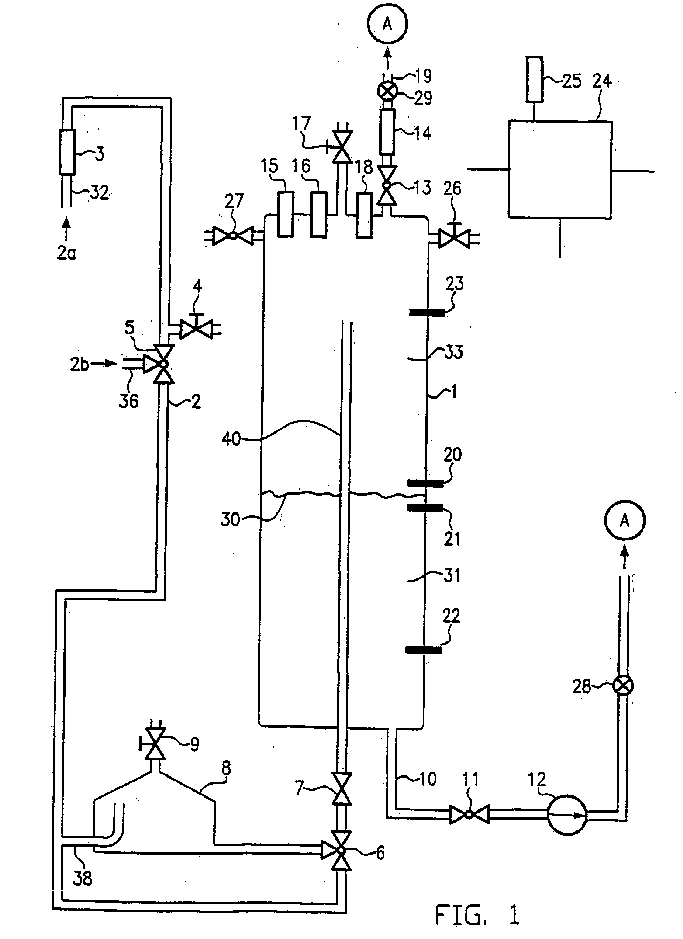

Die Erfindung wird anhand eines luftatmenden Öltransformators erläutert. Fig. 1 zeigt die Messeinrichtung schematisch. Die Anschlüsse 2a und 2b führen zum Flüssigkeitstank 48 des luftatmenden Öltransformators. Im Transformatorkessel 50 ist beispielsweise der Wicklungskörper des Öltransformators angeordnet. Ein solcher Öltransformator weist in bekannter Art ein Ausdehnungsgefäß A auf, das in der Figur nur als Anschlussoption gezeigt ist und höher als die erfindungsgemäße Messeinrichtung angeordnet ist. Das Ausdehnungsgefäß dient dazu, die Volumenvergrößerung des Öls im Transformatorkessel 50 im Betriebszustand aufgrund der vom Wicklungskörper abgegebenen Verlustwärme zu kompensieren. Das Ausdehnungsgefäß A, auch Ausdehner genannt, ist ein nach oben geöffnetes, atmosphärischem Druck ausgesetztes Gefäß, das über die Steigleitung 54 mit dem Kessel 50 verbunden ist, und in dem sich der Ölspiegel zwischen einer oberen und unteren Marke befinden muß. Der Abstand vom Fuße des Transformationskessels bis zum Ölspiegel im Ausdehner beträgt in der Regel 5 m und mehr. Die statische Säule des Öls im Ausdehnungsgefäß A ist daher proportional dem Druck im Transformatorkessel 50. Der Transformatorkessei weist ein Volumen von wenigen m³ bis etwa 100 m³ auf. Im Ausdehnungsgefäß A befinden sich etwa 3% bis 8% des Öls. Die Temperatur des Betriebsöls beträgt im Kessel etwa 35° bis 90° C. Das Öl im Ausdehnungsgefäß A kann dem Kesesl 50 über die Steigleitung teilweise wieder zurückgeführt werden, wenn beispielsweise die Öltemperatur wieder absinkt. Während des Betriebs unterliegt das Öl in der Steigleitung zwischen dem Kessel und Ausdehner der Konvektion.

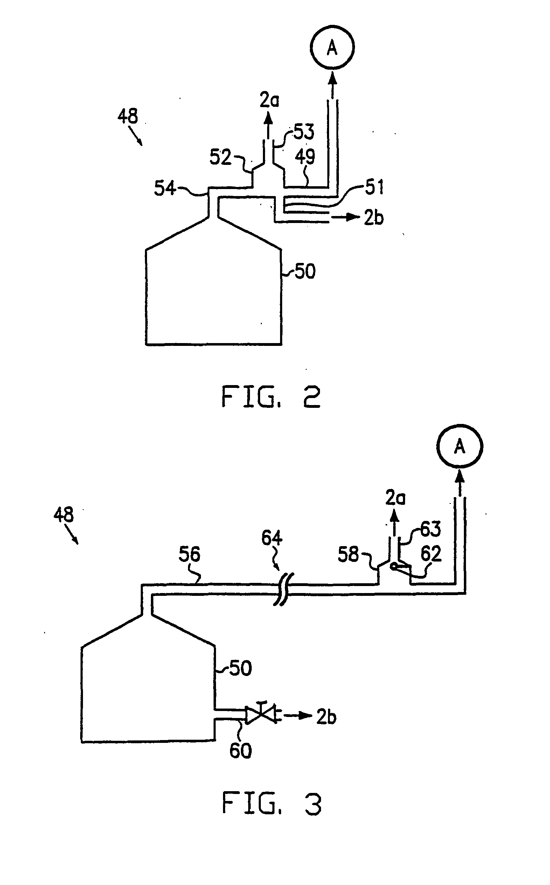

Fig. 2 zeigt schematisch eine mögliche Anschlussvariante. In der Steigleitung 54, die aus dem oberen Bereich des Flüssigkeitstankes 50 kommt, ist ein Gassammelstutzen 52 integriert. Die Entlüftung 53 des Stutzens ist mit dem Anschluss 2a der in Fig. 1 gezeigten Vorrichtung verbunden, während die Steigleitung 49 weiter zum Ausdehnungsgefäß A des Transformators führt. Der Ausgang 51 des Gassammelstutzens 52 führt zum Anschluss 2b der Fig. 1.

Fig. 3 zeigt schematisch eine andere Anschlussvariante. Der Flüssigkeitstank 48 mit dem Kessel 50 ist in bekannter Weise über eine Steigleitung 56 mit dem Ausdehnungsgefäß A verbunden. In der Steigleitung befindet sich ein Gassammelrelais, z.B. ein Buchholzrelais 58. Innerhalb des Relais 58 ist der Schwimmer 62 angedeutet.

Durch die Bruchlinien 64 ist angedeutet, dass mehrere Bögen und Verzweigungen in der Steigleitung 56 vorhanden sein können und das Buchholzrelais 58 nicht in unmittelbarer Nähe zum Flüssigkeitstank 48 angeordnet sein muss. Die Entlüftung 63 des Buchholzrelais 58 wird mit dem Anschluss 2a der Vorrichtung der Fig. 1 verbunden. Der Anschluss 2b der Vorrichtung der Fig. 1 wird mit dem Kessel 50 z.B. an einer vorhandenen Ölprobeentnahmestelle 60 des Flüssigkeitstankes 48 verbunden.

Sowohl in Fig. 2, als auch in Fig. 3 sind nur die zum Verständnis der Erfindung notwendigen Komponenten des flüssigkeitsgefüllten Transformators dargestellt.

Bei der in Fig. 1 gezeigten Messvorrichtung ist die Zuleitung 2b, zum Zuführen des Öls im Separiergefäß 1 unterhalb des in Fig. 3 gezeigten Buchholzrelais, bzw. unterhalb der Entlüftung 53, angeordnet. Die Zuleitung 2a dient zur Zuführung von in Öl ungelöstem Gas in das Separiergefäß 1. Vorzugsweise ist das Separiergefäß 1 im mittleren oder unteren Bereich des Flüssigkeitstanks 50 angeordnet. Somit kann aufgrund des Drucks der über den Zuführleitungen 2a, 2b ruhenden Ölsäule das Öl sowohl über die Zuleitungen 2a und 2b bei geöffneten Ventilen in das Separiergefäß 1 fließen. Da die Zuleitung 2a oberhalb der Entlüftung 53 bzw. 63 liegt, kann ungelöstes Gas, das nach oben steigt, dem Separiergefäß über die Zuführleitung 2a zugeführt werden.

Fig. 1 zeigt das im Wesentlichen zylindrisches abgeschlossenes Separiergefäß 1, in das von unten eine Zulaufkapillare 2 bis in den oberen Teil führt, der mit 33 bezeichnet ist und im Wesentlichen den Gasraum des Separiergefäßes darstellt und der unterhalb des Ausdehnungsgefässes angeordnet ist. Im Separiergefäß befindet sich im Normalbetrieb Flüssigkeit 31 mit einem Flüssigkeitsspiegel 30. Über ein Dreiwegeventil ist die Zuführung 2b mit einer Zuführung 36 verbunden, die im Betrieb den Ölzugang 2b bildet. Am dritten Weg des Dreiwegeventils 5 ist die Zuführung 32 angeschlossen, in der sich wie dargestellt in einem Steigleitungsbereich ein Füllstandssensor 3 befindet Die Zuführung 32 wird wie zuvor beschrieben an der Entlüftung des Flüssigkeitstanks oder an den Ausgang eines Gassammelrelais angeschlossen, das in der Regel bei luftatmenden Öltransformatoren vorhanden ist Ein solches Gassammelrelais ist z.B. ein Buchholzrelais zur Anzeige von ungelösten Gasen. Auf diese Weise wird eine Gaszuführung 2a für die Messeinrichtung gebildet. In der Zuführung 32 befindet sich ein

Ausgang mit einem Sperrhahn 4. Bevor die Zuführung 2 in das Separiergefäß 1 eintritt, passiert sie ein Regelventil 7. In einer Verzweigungsleitung der Zuführung 2 befindet sich ein Gassammelgefäß 8 mit einem Entlüftungssperrhahn 9. Die Zuführung in dieses Gassammelgefäß 8 ist mit 38 bezeichnet. Der Ausgang des Gassammelgefäßes 8 mündet über ein Dreiwegeventil 6 wieder in die Zulaufkapillare 2.

Der Teil 40 der Zulaufkapillare 2 innerhalb des Separiergefäßes erstreckt sich bis in den oberen Bereich oberhalb des Flüssigkeitsspiegels 30.

Das Separiergefäß 1 besitzt im Deckel einen Drucksensor 15, einen oder mehrere messgasverbrauchsfrei arbeitende Sensoren 16, einen Sperrhahn 17, sowie einen Höhenstandssensor 18. Der messgasverbrauchsfrei arbeitende Sensor 16 ist z.B. ein zur Analyse der gelösten Gase modifizierter Wärmeleitungssensor in Kombination mit selektiven IR-Sensoren. Der Höhenstandssensor 18 ist auf vorgegebene Höhenmarke 20, 21, 22, 23 des Separiergefäßes 1 eingestellt. Vom Deckel des Separiergefäßes 1 führt eine Spülkapillare 19, die ein Schaltventil 13 und ein Rückschlagventil 29 enthält, in den Gasraum des Ausdehnungsgefäßes A des Transformators. In der Spülgaskapillare 19 können ergänzende alternativ arbeitende Messsensoren 14 angeordnet sein.

Am Kopf des Separiergefäßes 1 befindet sich ein Sperrhahn bzw. Septum 26 und ein Schaltventil 27.

Vom Boden des Separiergefäßes 1 führt eine Ablauflcapillare 10 mit einem Schaltventil 11 und einer Pumpe 12 in das Ausdehnungsgefäß A des Transformators. Stromabwärts der Pumpe 12 ist ein Rückschlagventil 28 eingefügt.

Alle genannten Sensoren, Ventile und die Höhenmarken des Separiergefäßes 1 sowie die Pumpe 12 sind über entsprechende Leitungen mit einer Steuer- und Auswerteeinheit 24 verbunden, die nur schematisch dargestellt ist. Die Auswerteeinheit 24 kann zusätzlich mit einem Drucksensor 25 zur Messung des Umgebungsdrucks ausgestattet sein. Schließlich kann die Auswerteeinheit 24 mit dem Signalkreis eines Gassammelrelais 58 verschaltet sein, für den Fall, dass Anschluss 2a mit einem solchen Gassammelrelais, z.B. einem Buchholzrelais 58 verbunden ist.

Die Steuer- und Auswerteeinheit 24 übernimmt dabei unter anderem die Funktion eines Regelkreises. Als Regelgröße dient die Flüssigkeitsspiegelhöhe, die mit Hilfe des Höhenstandssensors 18 überwacht wird. Bei Überschreiten einer vorbestimmten Höhenmarke wird über die Steuer- und Auswerteeinheit 24 die Leistung der Pumpe 12 erhöht bzw. das Ventil 11 geöffnet. Bei Unterschreiten einer vorbestimmten Höhenstandsmarke wird die Pumpe 12 abgeschaltet bzw. das Ventil 11 geschlossen, so dass der Füllstand wiederum steigt.

Im Folgenden wird der Betrieb der erfindungsgemäßen Messeinrichtung an einem Beispiel erläutert, bei dem Anschluss 2a mit der Entlüftung eines Buchholzrelais 58 verbunden ist und Anschluss 2b mit dem Kessel 50 des Öltransformators, wie es in Fig. 3 gezeigt ist. Bekanntermaßen stellen Buchholzrelais seit langem ein wesentliches Element zur Überwachung und zum Schutz von ölgefüllten Transformatoren im Betrieb dar. Durch einen Fehler im Transformator erzeugte, nicht gelöste Zersetzungsgase sammeln sich in dem Buchholzrelais 58, welches bei Überschreitung einer bestimmten Gasmenge Alarm auslöst, oder gegebenenfalls eine Abschaltung des Transformators verursacht Sowohl das Signal zum Auslösen eines Alarms als auch das Signal zum Abschalten des Transformators werden in der Regel mechanisch durch entsprechende Schwimmer 62 verursacht. Ebenso können jedoch auch andere, z.B. elektronische Schaltmechanismen vorgesehen sein.

Die im Folgenden beispielhaft angegebenen Zeitangaben und Parameter gelten für ein Messgerät, das die folgenden vorteilhaften Maße aufweist Der Durchmesser der Kapillaren 2, 10, 19, 32, 36 ist kleiner oder gleich 4 mm. Das Volumen des Separiergefäßes 1 beträgt etwa 300 ml, wobei die Höhe des Separiergefäßes kleiner oder gleich 50 cm ist. Das Volumen des Gassammelgefäßes 8 beträgt etwa 50 ml. Die Höhenmarken 20 und 21 sind etwa mittig in dem Separiergefäß angeordnet und haben einen Abstand von etwa 3 mm. In der Regel beträgt die Temperatur des Separiergefäßes 35° bis 90°C. Die Installation der Messeinrichtung kann z.B. im unteren oder im mittleren Bereich des Kessels 50 erfolgen.

Die erfindungsgemäße Messeinrichtung muss vor dem Start gefüllt werden. Im Ausgangszustand sind alle Ventile geschlossen. Anschluss 2a wird an die Entlüftung des Buchholzrelais 58 angeschlossen. Der Sperrhahn 4 wird zur Entlüftung kurzzeitig geöffnet Entweder manuell oder mit Hilfe der Steuer- und Auswerteeinheit 24 werden die Dreiwegeventile 5 und 6 in Längsrichtung und die Ventile 7 und 13 geöffnet und der Höhenstandssensor 18 wird zugeschaltet Das Separiergefäß 1 füllt sich über die Leitung 32, 2 bis zur Höhenmarke 20 mit 01. Das Separiergefäß wird zunächst über die Zuführleitung 2a gefüllt, um sicherzustellen, dass auch die Zuführleitung 2a, 32, die dann als Zuführleitung für ungelöstes Gas verwendet wird, entlüftet ist. Das Separiergefäß könnte jedoch auch gleich über die Zuführleitung 2b, 36 gefüllt werden. Die Steuer- und Auswerteeinheit 24 öffnet daraufhin das Ventil 11 und schaltet die Pumpe 12 ein, die mit dem Höhenstandssensor 18 derart verkoppelt ist, dass der Ölspiegel zwischen den Höhenmarken 20 und 21 gehalten wird. Nach einer Einlaufzeit von z.B. 5 Minuten schaltet das Dreiwegeventil 5 auf Querrichtung. Da Anschluss 2b mit dem Kessel des Flüssigkeitstanks des Transformators verbunden ist, fließt dementsprechend nun über die Leitung 36, 2 Kesselöl in das Separiergefäß 1 ein. Das Öl kann aufgrund des herrschenden Überdrucks entsprechend der Ölsäule im Ausdehnungsgefäß über die Zuführleitungen 2a, 2b in das Separiergefäß fließen. Der durch die Pumpe 12 erzeugte Unterdruck saugt zudem das Öl in den Zuführleitungen an. Nach z.B. weiteren 10 Minuten schaltet das Dreiwegeventil 6 auf Querrichtung, so dass das Öl das Gassammelgefäß 8 durchfließt. Der Sperrhahn 9 wird kurzzeitig zur Entlüftung geöffnet und wieder verschlossen.

Im Anschluss daran werden auf die Sperrhähne 9 und 17 automatische Gasentnahmepatronen montiert und die Hähne geöffnet. Die Gasentnahmepatronen werden auf konventionelle Weise über entsprechende Leitungen mit der Steuer- und Auswerteeinheit 24 verbunden.

Der Mess- und Auswerteprozess kann nun gestartet werden. Entweder manuell oder mit Hilfe der Steuer- und Auswerteeinheit 24 wird das Dreiwegeventil 6 wieder auf Längsrichtung geschaltet und der Füllstandssensor 3, der Druckmesssensor 15, der modifizierte Wärmeleitungssensor 16 in Kombination mit selektiven IR-Sensoren und die optionale zusätzliche Messeinrichtung 14 werden zugeschaltet

Der Ölfluss durch das Separiergefäß 1 ist abhängig von der Ölspiegelhöhe im Ausdehnungsgefäß A und vom Gassättigungsgrad des Öles. Zur Vergleichmäßigung ist das Regelventil 7 vorgesehen, welches z.B. auf 4 Liter pro Stunde eingestellt wird. Bei den angegebenen Parametern ergeben sich Einstellzeit für den Gleichgewichtsdruck in dem Gasraum des Separiergefäßes 1 von etwa 2 Stunden, wobei diese Einstellzeit unter anderem von der Gestaltung der Zulaufkapillare 2 abhängt. Während der Einstellung des Gleichgewichtsdrucks fließt kontinuierlich Öl über die Zulaufleitungen 2b, 36 dem Separiergefäß zu und wird von der Pumpe 12 abgepumpt, um den Füllstand zwischen den Markierungen 21 und 20 zu halten. Das abgepumpte Öl wird dann wieder dem Ausdehnungsgefäß rückgeführt, und somit auch wieder dem Kessel 50, so dass ein geschlossener Kreislauf entsteht.

Nach Einstellung des Gleichgewichtdrucks ändern sich die Signale des Druckmesssensors 15 und des Sensors 16 nicht mehr. Im Gasraum 33 des Separiergefäßes 1 befindet sich das Gas der Flüssigkeit 31, woher sich die Gasraumgehalte nach dem Henry-Daltonschen Gesetz bestimmen. Die Signale des Druckmesssensors 15 und des Sensors 16 in Kombination mit selektiven IR-Sensoren können direkt zur Gasanalyse eingesetzt werden. So kann aus den Druckwerten der Gassättigungsgrad bestimmt werden und bei einem entsprechend modifizierten Wärmeleitfähigkeitssensors 16 in Kombination mit selektiven IR-Sensoren die Zusammensetzung der gelösten Gase in dem Öl bestimmt und/oder kontinuierlich überwacht werden.

Es können nun verschiedene Prozesse durchgeführt werden. Zuerst wird die Kalibrierung beschrieben. Die Steuer- und Auswerteeinheit 24 schließt das Schaltventil 11 und schaltet die Pumpe 12 ab. Daraufhin steigt der Ölspiegel 30 bis zur Höhenmarke 23. Ist diese erreicht, wird automatisch von der Steuer- und Auswerteeinheit 24 oder manuell das Schaltventil 27 geöffnet und Eichgas eingespült. Dabei ist das Schaltventil 13 geöffnet, so dass kein zusätzlicher Überdruck erzeugt wird. Die Zusammensetzung und der Druck des Eichgases sind bekannt, so dass aus den Signalen des Drucksensors 15 und des Wärmeleitfähigkeitssensors 16 Eichwerte festgelegt werden können. Jetzt kann das Schaltventil 13 wieder geschlossen werden.

Nach Abschluss der Kalibrierung wird manuell oder über die Steuer- und Auswerteeinheit 24 das Schaltventil 11 wieder geöffnet und die Pumpe 12 gestartet, so dass der Ölspiegel wieder in den Regelbereich der Höhenmarke 20 bzw. 21 gelangt.

Zur Entnahme einer Gasprobe wird wie folgt verfahren. Zuerst wird der Gleichgewichtsdruckwert des Sensors 15 gespeichert. Die Pumpe 12 wird abgeschaltet und das Schaltventil 11 geschlossen. Nach Abschalten der Pumpe steigt der Ölspiegel in dem Separiergefäß 1. Bei Erreichen der Höhenmarke 23 ist ein ausreichender Druck, von z.B. 1,2•10⁵ Pa erreicht, so dass über den Sperrhahn bzw. das Septum 26 eine Gasprobe entnommen werden kann. Alternativ kann über den Sperrhahn 17 eine automatische Gasentnahme in die aufgeschraubte Gasentnahmepatrone durchgeführt werden. Das entnommene Gas kann analysiert werden und gibt Informationen über den Anteil, die Konzentration und die Zusammensetzung des in Öl gelösten Gases.

Schließlich können bei Bedarf auch mit zusätzlichen anderen Messsensoren 14 andere Analysen durchgeführt werden. Dazu wird abweichend von dem beschriebenen Entnahmeprozess keine Gasprobe am Sperrhahn 26 oder dem Sperrhahn 17 entnommen, sondern bei Erreichen eines ausreichenden Drucks im Separiergefäß 1 das Ventil 13 geöffnet und nach der Messung mit dem Sensor 14 wieder geschlossen.

Nach der Probenentnahme wird über die Steuer- und Auswerteeinheit 24 oder manuell das Schaltventil 11 wieder geöffnet und die Pumpe 12 gestartet, so dass der Ölspiegel in den Regelbereich der Höhenmarken 20, 21 gelangt

Da messbare Änderungen im Öl des Flüssigkeitstanks des Transformators langzeitig eintreten, d.h. auf einer Zeitskala größer oder gleich einem Tag, können mit Hilfe der Messeinrichtung die im Öl gelösten Gase quasi-kontinuierlich überwacht werden, wenn die Messung einmal oder mehrmals am Tag durchgeführt wird. Aufgrund der großen Zeitskala ist es auch unproblematisch, dass kurzzeitig der Gleichgewichtszustand gestört wird.

Soll eine Dauerüberwachung durchgeführt werden, so wird die Entnahme periodisch durchgeführt. Dabei wird jeweils nach Durchführung einer Messung abgewartet, bis sich wieder der Gleichgewichtszustand eingestellt hat, d.h. etwa ein bis zwei Stunden.

Die Vorrichtung gestattet also eine ggf. kontinuierliche Messung des Gassättigungsgrades über den Druckmesssensor 15, eine Aussage über die Gaszusammensetzung über den modifizierten Wärmeleitfähigkeitssensor 16 in Kombination mit selektiven IR-Sensoren und eine Vollanalyse des gelösten Gases mit Hilfe der entnommenen Gasproben.

Im Folgenden wird der Verfahrensgang beim zusätzlichen Auftreten ungelöster Gase geschildert.

Im Normalbetrieb ist das Dreiwegeventil 5 zur Verbindung der Anschlüsse 36 und 2 geschaltet Das Ventil 4 ist geschlossen. 2a ist, wie beschrieben, bei dieser Ausführungsform an den Gassammelraum eines Buchholzrelais 58 angeschlossen. Ungelöstes Gas, das sich in diesem Gasraum gesammelt hat, verdrängt Öl aus der Zuleitung 32, so dass der Füllstandsensor 3 anspricht und ein Signal an die Auswerte- und Steuereinheit 24 gibt.

Beim Ansprechen des Füllstandssensors 3 wird von der Steuer- und Auswerteeinheit 24 der Vergleichszustand für gelöste Gase im Separiergefäß 1 kontrolliert bzw. hergestellt Ist z.B. während eines längeren Zeitraumes, z.B. 2 Stunden, das Ventil 11 nicht betätigt worden, so kann von einem Gleichgewichtszustand in dem Separiergefäß 1 ausgegangen werden, da keine Kalibrierung oder Entnahmevorgänge stattgefunden haben. Ist jedoch innerhalb eines Zeitraums von etwa 2 Stunden der Gleichgewichtszustand durch eine Entnahme gestört gewesen, so hat sich in der Regel der Gleichgewichtsdruck in dem Separiergefäß 1 noch nicht wieder eingestellt. Die Auswerte- und Steuereinheit 24 bildet dann den Quotienten aus dem aktuell gemessenen Druckwert des Sensors 15 und des vor der letzten Kalibrierung bzw. Entnahme gespeicherten Druckwerts. Mit dem so gebildeten Verdünnungsfaktor ist ein Maß für die Abweichung von dem Gleichgewichtszustand bekannt. Mit diesem Verdünnungsfaktor werden die Messwerte der Sensoren korrigiert.

Nur wenn kurz vor dem Ansprechen des Füllstandssensors 3 eine Eichgaszuführung zur Kalibrierung des Ventils 27 stattgefunden hat, muss mit der weiteren Durchführung des Verfahrens 1 bis 2 Stunden gewartet werden, bis sich das Eichgas aus dem Separierraum ausreichend verflüchtigt hat

Nach Ansprechen des Füllstandsensors 3 überprüft die Steuer- und Auswerteeinheit zur Sicherheit, ob der Flüssigkeitsspiegel 30 zwischen den Höhenmarken 20 und 21 ist. Vor Fortführung des Verfahrens wird gegebenenfalls solange gewartet, bis durch Pumpen mit Hilfe der Pumpe 12 der Flüssigkeitsspiegel 30 wieder zwischen den Höhenmarken 20 und 21 angelangt ist.

Spricht der Füllstandssensor 3 an, so ist zumindest eine geringe Konzentration von ungelösten Gasen vorhanden. Spricht zusätzlich ein Gassammelrelais z.B. das Buchholzrelais 58 des Öltransformators an, so sind größere Konzentrationen ungelösten Gases vorhanden und es wird vorteilhafterweise wie folgt verfahren. Das Dreiwegeventil 5 bleibt zunächst in der Stellung, in der es im Flüssigkeitstank des Öltransformators über die Leitungen 36 und 2 mit dem Separiergefäß 1 verbindet und die Leitung 32 abschließt. So kann eine Probenentnahme des gelösten Gases vorgenommen werden, wie es oben beschrieben ist.

Nachdem der Ölspiegel wieder in den Regelbereich zwischen den Marken 20 und 21 angelangt ist, schaltet das Dreiwegeventil 5 auf Längsrichtung und verbindet den Zulauf 32 mit der Zuführkapillare 2. Das Dreiwegeventil 6 schaltet auf Querrichtung, so dass auch das Gassammelgefäß 8 zugeschaltet ist. Da sich in dem Zulauf 32 ungelöste Gase befinden, werden diese nun durch die Zulaufkapillare 2 zuerst in das Gassammelgefäß 8 und dann in das Separiergefäß 1 transportiert Dabei verändern sich die Zeitfunktionen der Sensoren 15 und 16. Aus der Änderung der Zeitfunktionen lässt sich die Herkunft der ungelösten Gase ableiten. Durch das Einströmen der ungelösten Gase in das Separiergefäß 1 senkt sich der Flüssigkeitsspiegel 30. Erreicht der Ölspiegel die untere Höhenmarke 22, schließt das Ventil 11 und die Pumpe 12 wird ausgeschaltet. Nun wird das Ventil 13 geöffnet und das ungelöste Gas, welches in das Separiergefäß 1 gelangt ist, entweicht über die Spülgaskapillare 19. Liegt am Füllstandssensor 3 kein Signal mehr an, so ist das ungelöste Gas über die Zuführung 32 und 2 aus dem Flüssigkeitstank des Transformators herausgetrieben, d.h. an dem Füllstandssensor 3 befindet sich wieder Flüssigkeit. Jetzt wird von der Steuer- und Auswerteeinheit 24 oder manuell die automatische Gasentnahmepatrone oberhalb des Gassammelrelais 8 auf dem Sperrhahn 9 ausgelöst. In dem Gassammelgefäß 8 hat sich ungelöstes Gas angesammelt, so dass in der Gasentnahmepatrone auf dem Sperrhahn 9 ungelöstes Gas aufgefangen wird.

Da die Spülgaskapillare 19, die mit dem Ausdehnungsgefäß in Verbindung steht, geöffnet ist, steigt in dem Separiergefäß 1 der Ölspiegel wieder an. Um die ungelösten Gase möglichst verständig aus dem Separiergefäß 1 zu entfernen, lässt die Steuer- und Auswerteeinheit 24 den Ölspiegel 30 bis zur Höhenmarke 23 ansteigen. Daraufhin wird das Dreiwegeventil 5 wiederum auf Querrichtung geschaltet, so dass wieder die Flüssigkeit aus dem Kessel 50 des Öltransformators über den Weg 36, 2 in das Separiergefäß 1 gelangt. Das Dreiwegeventil 6 schaltet wieder in Längsrichtung, so dass kein Durchfluss mehr durch das Gassammelgefäß 8 stattfindet. Durch Einschalten der Pumpe 12 und Öffnen des Ventils 11 bei gleichzeitigem Schließen des Ventils 13 wird erreicht, dass der Ölspiegel 30 wieder in den Regelbereich zwischen den Höhenmarken 20 und 21 gelangt. Nach Ablauf einer gewissen Zeit, z.B. 2 Stunden, hat sich die Gleichgewichtsphase im Separiergefäß 1 wieder eingestellt.

Die Sperrhähne 9 und 17 werden manuell oder durch die Steuer und Auswerteeinheit 24 geschlossen und die automatischen Gasentnahmepatronen, die daran befestigt waren, entfernt Ihr Inhaltsgas steht nun für gaschromatographische Vollanalysen bereit Zur Einstellung des Normalzustandes muss noch das Gassammelgefäß 8 über den Sperrhahn 9 entlüftet werden.

Für die gaschromatographischen Vollanalysen ist es dabei von besonderem Vorteil, dass das Gleichgewichtsgas, das an der Gasentnahmepatrone 17 entnommen wurde, nahezu gleichzeitig mit dem ungelösten Gas, das an dem Sperrhahn 19 entnommen wurde, abgenommen wurde. Aufwendige Umrechnungsarbeiten aufgrund einer zeitlichen Verzögerung zwischen der Entnahme des Gleichgewichtsgases und des ungelösten Gases sind dementsprechend nicht nötig.

Beschrieben wurde der Fall, dass der Füllstandssensor 3 anspricht und zusätzlich ungelöste Gase mit Hilfe des am Transformator vorhandenen Buchholzrelais 58 angezeigt werden. Es kann jedoch auch der Fall auftreten, dass der Füllstandssensor 3 anspricht, ohne dass gleichzeitig das Buchholzrelais ein Signal gibt. In diesem Fall sind nur sehr geringe Mengen ungelöster Gase vorhanden und es wird ein abweichender Prozess durchgeführt. Durch Schalten des Ventils 5 auf Längsrichtung werden die Zuleitungen 32 und 2 miteinander verbunden, so dass die ungelösten Gase in den Gasraum 33 des Separiergefäßes 1 eintreten. Dadurch werden die Zeitfunktionen der Sensoren 15 und 16 geändert und wie beschrieben zur Herkunftsbestimmung der ungelösten Gase eingesetzt Bei dieser alternativen Vorgehensweise kann auch bei geringen Konzentrationen ungelöster Gase zumindest deren Herkunft bestimmt werden.

Mit der erfindungsgemäßen Vorrichtung ist es also zum einen möglich, quasi-kontinuierlich den Gleichgewichtszustand der im Öl gelösten Gase zu überwachen. Dazu sind Manipulationsvorrichtungen, z.B. die Entnahmevorrichtung 26 bzw. 17 vorgesehen. Das abgeschlossene Separiergefäß 1 bildet einen Gleichgewichtsgasraum, der auch dann noch Gleichgewichtsgas enthält, wenn bereits ungelöste Gase an dem Anschluss 2a anliegen. So ist es möglich, dass nahezu gleichzeitig die Parameter des Gleichgewichtsgases und die Parameter der ungelösten Gase bestimmt werden. Indem vor dem Einleiten der ungelösten Gase in den Gasraum des Separiergefäßes 1 bereits die Gleichgewichtsparameter des der im Öl gelösten Gase bestimmt worden sind, ist eine Störung der Sensorfunktion durch die ungelösten Gase, die im Anschluss daran eingelassen werden, unerheblich.

Die Messeinrichtung für mit Flüssigkeit gefüllte Hochspannungsgeräte kann an vorhandenen Probestellen für Flüssigkeiten und ungelöste Gase angeschlossen werden. Es wird kontinuierlich eine Gleichgewichtsgasphase für die gelösten Gase bereitgestellt, in der messgasverbrauchsfreie Sensoren arbeiten können und die periodisch mittels des Flüssigkeitsspiegels für Kalibrierungen, Gasentnahmen oder Ausspülungen für ergänzende Sensormessungen druckverändert werden kann. Die Zuführung von auftretenden ungelösten Gasen kann direkt und schnell nach ihrem Erscheinen erfolgen und mit Hilfe gesonderter automatischer Gasentnahmepatronen einer externen Vollanalyse zugeführt werden. Dazu kann eine nahezu gleichzeitige entnommene Probe des Gleichgewichtsgases als Referenz dienen.

## Patentansprüche

1. Verfahren zur Überwachung von gelösten Gasen in flüssigkeitsgefüllten Hochspannungsanlagen mit einem geschlossenen Separiergefäß (1), das über eine Zuführleitung (2, 36) in Verbindung mit dem Flüssigkeitstank (48) der Hochspannungsanlage steht, wobei der Flüssigkeitstank über eine Leitung (54) mit einem Ausdehnungsgefäß (A) verbunden ist, mit folgenden Schritten:
a) Füllen des Separiergefäßes (1) über die Zuführleitung (2, 36) mit der Flüssigkeit aus dem Flüssigkeitstank (48), wobei die Flüssigkeit aufgrund der statischen Säule der Flüssigkeit über der Zuführleitung (2, 36) aus dem Flüssigkeitstank in das Separiergefäß fließen kann,
b) Aufrechthalten eines Gleichgewichtszustandes zwischen Flüssigkeit (31) und Gasphase im Separiergefäß (1), indem der Flüssigkeitsspiegel (30) durch Abpumpen der Flüssigkeit mit einer Pumpe (12) auf einer vorbestimmten Höhe gehalten wird, wobei die abgepumpte Flüssigkeit dem Flüssigkeitstank (48) oder dem Ausdehnungsgefäß (A) rückgeführt wird,
c) Kurzzeitiges Verlassen des Gleichgewichtszustandes durch Unterbrechen des Pumpvorganges oder Verringern der Pumpleistung, um in dem Separiergefäß (1) den Flüssigkeitsspiegel zu erhöhen und
d) Analyse des Gases oberhalb des Flüssigkeitsspiegels (30) in dem Separiergefäß (1) entweder durch
d₁) Entnahme einer Gasprobe aus dem Gas (33) oberhalb des Flüssigkeitsspiegels (30) und/oder durch
d₂) einen messgasverbrauchsfreien Sensor (15, 16) im Gasraum (33) des Separiergefäßes (1) oberhalb des Flüssigkeitsspiegels (30).

2. Verfahren nach Anspruch 1, bei dem in Schritt d₁ die Gasprobe manuell an einem Septum bzw. Sperrhahn (26) entnommen wird.

3. Verfahren nach Anspruch 1, bei dem in Schritt d₁ die Gasprobe automatisch mit Hilfe einer Gasentnahmepatrone entnommen wird.

4. Verfahren nach Anspruch 1, bei dem in Schritt d₁ Gasprobe über ein Ventil (13) entnommen wird, das mit wenigstens einem Sensor (14) zur Gasanalyse in Verbindung steht.

5. Verfahren nach Anspruch 1, wobei die Schritte c) und d) periodisch wiederholt werden.

6. Verfahren nach Anspruch 1, wobei zusätzlich zu den Schritten a bis d zur Überwachung von ungelösten Gasen folgende Schritte durchgeführt werden:
e) Zuführen von ungelösten Gasen über die zweite Zuführung (32) in den Gasraum (33) oberhalb des Flüssigkeitsspiegels (30) in dem Separiergefäß (1),
f) Überwachen der zeitlichen Änderung der Analysenwerte und
g) Auswerten der zeitlichen Änderung der Analysenwerte zur Herkunftsbestimmung des ungelösten Gases.

7. Verfahren nach Anspruch 6, bei dem die Analysenwerte Ergebnisse von Analysen einer oder mehrerer entnommener Gasproben umfassen.

8. Verfahren nach Anspruch 6 oder 7, bei dem die Analysenwerte die Signale von zumindest einem messgasverbrauchsfreien Sensor (15, 16) im Separiergefäß (1) oberhalb des Flüssigkeitsspiegels (30) umfassen.

9. Verfahren nach einem der Ansprüche 6 bis 8, bei dem
über eine Gasleitung (38), die von der Zuführung (2) abzweigt, ungelöste Gase in ein Gassammelgefäß (8) abgeführt werden, und
nach dem Schritt e) eine Gasprobe aus dem Gassammelgefäß (8) entnommen wird.

10. Verfahren nach einem der Ansprüche 6 bis 9, wobei das ungelöste Gas aus der Entlüftung (53) eines Gassammelstutzens (52) zugeführt wird, der direkt oberhalb des Flüssigkeitstanks (48) in einem Steigleitungsbereich (54) zwischen dem oberen Bereich des Flüssigkeitstanks und dem Separiergefäß (1) angeordnet ist und zur Ölzufuhr mit der ersten Zuführung (2, 36) verbunden ist.

11. Verfahren nach einem der Ansprüche 6 bis 9, bei dem das ungelöste Gas aus einem Gassammelrelais, z. B. einem Buchholzrelais (58), des Flüssigkeitstankes der Hochspannungsanlage entnommen wird.

12. Vorrichtung zur Überwachung von gelösten Gasen in flüssigkeitsgefüllten Hochspannungsanlagen, insbesondere zur Durchführung eines Verfahrens nach Anspruch 1, mit
- einem geschlossenen Separiergefäß (1),
- einer Zuführung (2, 36) zum Zuführen der Flüssigkeit aus einem Flüssigkeitstank in das Separiergefäß, die mit dem Flüssigkeitstank (48) der Hochspannungsanlage in Verbindung steht und in das Separiergefäß (1) in einer Höhe mündet, die oberhalb des Flüssigkeitsspiegels (30) im Separiergefäß (1) liegt, wenn dort ein Gleichgewichtszustand herrscht, wobei der Flüssigkeitstank über eine Leitung (54) mit einem Ausdehnungsgefäß (1) verbunden ist, und die Flüssigkeit aufgrund der statischen Säule der Flüssigkeit über der Zuführung aus dem Flüssigkeitstank in das Separiergefäß fließen kann,
- einer Pumpe (12), die mit dem Separiergefäß (1) an einer Stelle verbunden ist, die unterhalb des Flüssigkeitsspiegels (30) im Separiergefäß (1) liegt,
- einer Leitung zum Rückführen der abgepumpten Flüssigkeit aus dem Separiergefäß (1) in den Flüssigkeitstank (48) oder das Ausdehnungsgefäß (A),
- zumindest einem Entnahmeventil (26, 17, 13) zur Entnahme von Gasproben oberhalb des Flüssigkeitsspiegels (30) im Separiergefäß (1), und
- einem Höhenstandmeßsystem (18, 20, 21) zur Detektion des Flüssigkeitsspiegels (30) im Separiergefäß (1).

13. Vorrichtung nach Anspruch 12, wobei das Höhenstandmeßsystem (18, 20, 21) mit der Pumpe (12) und/oder einem Ventil (11) zur Abkoppelung der Pumpe (12) vom Separiergerät (1) derart in Art eines Regelkreises verbunden ist, dass die Pumpe (12) und/oder das Ventil (11) in Abhängigkeit des Füllstandes im Separiergefäß (1) als Regelgröße steuerbar ist, so dass eine vorbestimmte Flüssigkeitsspiegelhöhe eingehalten wird.

14. Vorrichtung nach Anspruch 12 oder 13 mit einem Schaltventil (27) am Separiergefäß (1) oberhalb des Flüssigkeitsspiegels (30) zur Zuführung von Eichgas.

15. Vorrichtung nach einem der Ansprüche 12 bis 14, bei dem die Zuführung (2) von unten in das Separiergefäß (1) eingeführt ist und bis oberhalb des Flüssigkeitsspiegels (30) reicht, wenn ein Gleichgewichtszustand im Separiergefäß (1) herrscht.

16. Vorrichtung nach einem der Ansprüche 12 bis 15, mit wenigstens einem messgasverbrauchfreien Gassensor (16) im Separiergefäß (1) oberhalb des Flüssigkeitsspiegels (30) im Gleichgewichtszustand.

17. Vorrichtung nach einem der Ansprüche 12 bis 16 mit einem Druckmesssensor (15).

18. Vorrichtung nach einem der Ansprüche 12 bis 17 mit einer Spülgasleitung (19) zum Gasablassen aus dem Separiergefäß (1).

19. Vorrichtung nach Anspruch 18, mit einem Sensor (14) in der Spülgasleitung (19) zur Gasanalyse.

20. Vorrichtung nach einem der Ansprüche 12 bis 19, mit einer zweiten Zuführung (32) zur Zuführung ungelöster Gase, die das Separiergefäß (1) mit dem Flüssigkeitstank (48) verbindet und in einem Steigleitungsbereich einen Füllstandsensor (3) aufweist, und einer Ventileinrichtung (5) zur alternativen Auswahl der ersten (36) oder zweiten (32) Zuführung.

21. Vorrichtung nach Anspruch 20, bei der die Ventileinrichtung ein Dreiwegeventil (5) umfasst, dessen einer Anschluss mit einer Steigkapillare (2) zum Separiergefäß (1) und dessen zweiter bzw. dritter Anschluss mit der ersten (36) bzw. zweiten (32) Zuführung verbunden ist.

22. Vorrichtung nach Anspruch 20 oder 21, bei der die zweite Zuführung (32) zur Verbindung mit der Entlüftung (63) eines Gassammelrelais (58) des Flüssigkeitstankes der Hochspannungsanlage und die erste Zuführung (36) zum Anschluss an den Kessel (50) der Hochspannungsanlage geeignet ist.

23. Vorrichtung nach Anspruch 20 oder 21 mit einem Gassammelstutzen (52), dessen Entlüftung (53) mit der zweiten Zuführung (32) verbunden ist, wobei der Gassammelstutzen in einer Steigleitung (51, 54), die den oberen Bereich des Flüssigkeitstankes (48) mit der ersten Zuführung (36) verbindet, direkt oberhalb des Flüssigkeitstankes angeordnet wird.

24. Vorrichtung nach einem der Ansprüche 20 bis 23, mit einem Gassammelgefäß (8) in das eine Verzweigung (38) der zweiten Zuführung (2, 32) führt, mit einem Anschluss (9) im oberen Bereich zur Probeentnahme.

## Claims

1. Method for monitoring dissolved gases in liquid-filled high-voltage installations comprising a sealed separating vessel (1) communicating via a feeding pipe (2, 36) with the liquid tank (48) of the high-voltage installation, wherein the liquid tank is connected with an oil conservator (A) via a pipe (54), including the following steps:
a) filling the separating vessel (1) via the feeding pipe (2, 36) with the liquid from the liquid tank (48), wherein, due to the static column of the liquid above the feeding pipe (2, 36), the liquid can flow from the liquid tank into the separating vessel,
b) maintaining an equilibrium state between the liquid (31) and the gas phase in the separating vessel (1) by keeping the liquid level (30) at a predetermined height through pumping off the liquid with a pump (12), wherein the pumped off liquid is fed back to the liquid tank (48) or to the oil conservator (A),
c) short-term leaving the equilibrium state by interrupting the pumping action or by reducing the pumping performance so as to increase the liquid level in the separating vessel (1), and
d) analyzing the gas above the liquid level (30) in the separating vessel (1) either by
d₁) withdrawing a gas sample of the gas (33) from above the liquid level (30) and/or by
d₂) a sensor (15, 16) free of test gas consumption in the gas phase (33) of the separating vessel (1) above the liquid level (30).

2. Method according to claim 1, wherein in step d₁ the gas sample is withdrawn manually on a septum or stop valve (26).

3. Method according to claim 1, wherein in step d₁ the gas sample is withdrawn automatically by means of a gas withdrawal cartridge.

4. Method according to claim 1, wherein in step d₁ the gas sample is withdrawn via a valve (13) communicating with at least one sensor (14) for the gas analysis.

5. Method according to claim1, wherein steps c) and d) are periodically repeated.

6. Method according to claim 1, wherein, in addition to steps a to d for monitoring undissolved gases, the following steps are carried out:
e) feeding undissolved gases via the second feeding pipe (32) into the gas phase (33) above the liquid level (30) in the separating vessel (1),
f) monitoring the temporal change of the analyzed values, and
g) evaluating the temporal change of the analyzed values for determining the origin of the undissolved gas.

7. Method according to claim 6, wherein the analyzed values comprise results of analyses of one or more withdrawn gas samples.

8. Method according to claim 6 or 7, wherein the analyzed values comprise the signals from at least one sensor (15, 16) free of gas test consumption in the separating vessel (1) above the liquid level (30).

9. Method according to one of claims 6 to 8, wherein
undissolved gases are discharged into a gas collecting vessel (8) via a gas pipe (38) branching off from the feeding pipe (2), and
a gas sample is withdrawn from the gas collecting vessel (8) after step e).

10. Method according to one of claims 6 to 9, wherein the undissolved gas is fed from the ventilation assembly (53) of a gas collection fitting (52) arranged directly above the liquid tank (48) in a rising pipe portion (54) between the upper portion of the liquid tank and the separating vessel (1) and connected with the first feeding pipe (2, 36) for the supply of oil.

11. Method according to one of claims 6 to 9, wherein the undissolved gas is withdrawn from a gas collecting relay, e.g. a beech wood relay (58), of the liquid tank of the high-voltage installation.

12. Device for monitoring dissolved gases in liquid-filled high-voltage installations, particularly for performing a method according to claim 1, comprising
- a sealed separating vessel (1),
- a feeding pipe (2, 36) for feeding the liquid from a liquid tank into the separating vessel (1), which communicates with the liquid tank (48) of the high-voltage installation and runs into the separating vessel (1) at a height being above the liquid level (30) in the separating vessel (1) when an equilibrium state is prevailing therein, wherein the liquid tank is connected via a pipe (54) with an oil conservator (1) and, due to the static column of the liquid above the feeding pipe, the liquid can flow out of the liquid tank into the separating vessel,
- a pump (12) connected with the separating vessel (1) on a point underneath the liquid level (30) in the separating vessel (1),
- a pipe for feeding the pumped off liquid from the separating vessel (1) back into the liquid tank (48) or the oil conservator (A),
- at least one withdrawal valve (26, 17, 13) for withdrawing gas samples above the liquid level (30) in the separating vessel (1), and
- a height level measuring system (18, 20, 21) for detecting the liquid level (30) in the separating vessel (1).

13. Device according to claim 12, wherein the height level measuring system (18, 20, 21) is connected with the pump (12) and/or a valve (11) for uncoupling the pump (12) from the separating vessel (1) in a control-circuit-type such that the pump (12) and/or the valve (11) are controllable as controlled magnitude in response to the liquid level in the separating vessel (1), so that a predetermined liquid level height is observed.

14. Device according to claim 12 or 13, comprising an on-off valve (27) on the separating vessel (1) above the liquid level (30) for the feeding of calibration gas.

15. Device according to one of claims 12 to 14, wherein the feeding pipe (2) is introduced into the separating vessel (1) from underneath and reaches until above the liquid level (30) when an equilibrium state prevails in the separating vessel (1).

16. Device according to one of claims 12 to 15, comprising at least one gas sensor (16) free of test gas consumption in the separating vessel (1) above the liquid level (30) in the equilibrium state.

17. Device according to one of claims 12 to 16 comprising a pressure measuring sensor (15).

18. Device according to one of claims 12 to 17 comprising a purge-gas pipe (19) for discharging gas out of the separating vessel (1).

19. Device according to claim 18 comprising a sensor (14) in the purge-gas pipe (19) for the gas analysis.

20. Device according to one of claims 12 to 19, with a second feeding pipe (32) for feeding undissolved gases connecting the separating vessel (1) with the liquid tank (48) and comprising a liquid level sensor (3) in a rising pipe portion, and with a valve arrangement (5) for alternatively selecting the first (36) or the second (32) feeding pipe.

21. Device according to claim 20, wherein the valve arrangement comprises a three-way valve whereof (5) the one connection is connected with a rising capillary (2) to the separating vessel (1) and whereof the second or third connection is connected with the first (36) or second (32) feeding pipe.

22. Device according to claim 20 or 21, wherein the second feeding pipe (32) is suited to connect with the ventilation assembly (63) of a gas collecting relay (58) of the liquid tank of the high-voltage installation and the first feeding pipe (36) is suited to connect with the tank (50) of the high-voltage installation.

23. Device according to claim 20 or 21, comprising a gas collection fitting (52) whereof the ventilation assembly (53) is connected with the second feeding pipe (32), wherein the gas collection fitting is arranged in a rising pipe (51, 54), which connects the upper portion of the liquid tank (48) with the first feeding pipe (36), directly above the liquid tank.

24. Device according to one of claims 20 to 23, comprising a gas collecting vessel (8) into which leads a branch (38) of the second feeding pipe (2, 32), with a connection (9) in the upper portion for the withdrawal of the sample.

## Revendications

1. Procédé de contrôle de gaz dissous dans des installations haute tension remplies de liquide qui comprennent un séparateur fermé (1) relié par une conduite d'amenée (2, 36) au réservoir de liquide (48) de l'installation haute tension, lequel réservoir de liquide est relié par une conduite (54) à un réservoir d'expansion (A), avec les étapes qui consistent à :
a) remplir le séparateur (1) avec le liquide du réservoir de liquide (48) par l'intermédiaire de la conduite d'amenée (2, 36), le liquide pouvant s'écouler du réservoir de liquide vers le séparateur par la conduite d'amenée (2, 36) grâce à la colonne statique du liquide,
b) maintenir un état d'équilibre entre le liquide (31) et la phase gazeuse dans le séparateur (1) en maintenant le niveau de liquide (30) à une hauteur prédéfinie, grâce au pompage du liquide à l'aide d'une pompe (12), le liquide pompé étant ramené dans le réservoir de liquide (48) ou dans le réservoir d'expansion (A),
c) quitter temporairement l'état d'équilibre en interrompant le pompage ou en réduisant la puissance de pompage, pour augmenter le niveau de liquide dans le séparateur (1), et
d) analyser le gaz au-dessus du niveau de liquide (30) dans le séparateur (1) grâce
d₁) au prélèvement d'un échantillon du gaz (33) au-dessus du niveau de liquide (30) et/ou
d₂) à un capteur sans consommation de gaz de mesure (15, 16) dans l'espace de gaz (33) du séparateur (1), au-dessus du niveau de liquide (30).

2. Procédé selon la revendication 1, selon lequel lors de l'étape d₁ l'échantillon de gaz est prélevé manuellement au niveau d'un diaphragme ou d'un robinet d'arrêt (26).

3. Procédé selon la revendication 1, selon lequel lors de l'étape d₁ l'échantillon de gaz est prélevé automatiquement à l'aide d'une cartouche de prélèvement de gaz.

4. Procédé selon la revendication 1, selon lequel lors de l'étape d₁ un échantillon de gaz est prélevé grâce à une soupape (13) qui est reliée à au moins un capteur (14) pour l'analyse du gaz.

5. Procédé selon la revendication 1, selon lequel les étapes c) et d) sont répétées périodiquement.

6. Procédé selon la revendication 1, selon lequel en plus des étapes a à d on procède aux étapes suivantes pour contrôler des gaz non dissous :
e) amenée de gaz non dissous, par l'intermédiaire de la seconde conduite d'amenée (32), dans l'espace de gaz (33) situé au-dessus du niveau de liquide (30) dans le séparateur (1),
f) contrôle de la variation dans le temps des valeurs d'analyse, et
g) évaluation de la variation dans le temps des valeurs d'analyse pour définir l'origine du gaz non dissous.

7. Procédé selon la revendication 6, selon lequel les valeurs d'analyse comprennent les résultats d'analyses d'un ou plusieurs prélèvements de gaz.

8. Procédé selon la revendication 6 ou 7, selon lequel les valeurs d'analyse comprennent les signaux d'au moins un capteur sans consommation de gaz de mesure (15, 16) prévus dans le séparateur (1), au-dessus du niveau de liquide (30).

9. Procédé selon l'une des revendications 6 à 8, selon lequel
par l'intermédiaire d'une conduite de gaz (38) qui part de la conduite d'amenée (2), des gaz non dissous sont évacués dans un collecteur de gaz (8) et
après l'étape e) un échantillon de gaz est prélevé dans le collecteur (8).

10. Procédé selon l'une des revendications 6 à 9, selon lequel le gaz non dissous est amené à partir du dispositif de désaération (53) d'un tuyau collecteur de gaz (52) qui est disposé juste au-dessus du' réservoir de liquide (48), dans une zone de conduite montante (54) située entre la zone supérieure du réservoir de liquide et le séparateur (1), et qui est relié pour l'amenée de l'huile à la première conduite d'amenée (2, 36).

11. Procédé selon l'une des revendications 6 à 9, selon lequel le gaz non dissous est prélevé dans un relais collecteur de gaz, par exemple un relais Buchholz (58), du réservoir de liquide de l'installation haute tension.

12. Dispositif de contrôle de gaz dissous dans des installations haute tension remplies de liquide, en particulier pour la mise en oeuvre du procédé selon la revendication 1, comprenant
- un séparateur fermé (1),
- une conduite d'amenée (2, 36) pour amener le liquide d'un réservoir de liquide jusqu'au séparateur, qui est reliée au réservoir de liquide (48) de l'installation haute tension et qui débouche dans le séparateur (1) à une hauteur située au-dessus du niveau de liquide (30) dans ledit séparateur (1) lorsqu'il y a un état d'équilibre dans celui-ci, le réservoir de liquide étant relié par une conduite (54) à un réservoir d'expansion (A) et le liquide pouvant s'écouler du réservoir de liquide dans le séparateur, par la conduite d'amenée, grâce à la colonne statique du liquide,
- une pompe (12) qui est reliée au séparateur (1) à un endroit qui se trouve au-dessous du niveau de liquide (30) dans le séparateur (1),
- une conduite pour ramener dans le réservoir de liquide (48) ou dans le réservoir d'expansion (A), à partir du séparateur (1), le liquide pompé,
- au moins une soupape de prélèvement (26, 17, 13) pour le prélèvement d'échantillons de gaz au-dessus du niveau de liquide (30) dans le séparateur (1), et
- un système de mesure de niveau (18, 20, 21) pour détecter le niveau de liquide (30) dans le séparateur (1).

13. Dispositif selon la revendication 12, selon lequel le système de mesure de niveau (18, 20, 21) est relié à la manière d'un circuit régulateur à la pompe (12) et/ou à une soupape (11) pour le découplage de la pompe (12) et du séparateur (1), de sorte que la pompe (12) et/ou la soupape (11) sont aptes à être commandées en fonction du niveau de remplissage du séparateur (1), comme grandeur de réglage, de sorte qu'un niveau de liquide prédéfini est respecté.

14. Dispositif selon la revendication 12 ou 13 avec une soupape de commande (27) sur le séparateur (1), au-dessus du niveau de liquide (30), pour l'amenée de gaz d'étalonnage.

15. Dispositif selon l'une des revendications 12 à 14, avec lequel la conduite d'amenée (2) est introduite dans le séparateur (1) par le bas et va jusqu'au-dessus du niveau de liquide (30) lorsqu'un état d'équilibre règne dans le séparateur (1).

16. Dispositif selon l'une des revendications 12 à 15, avec au moins un capteur sans consommation de gaz de mesure (16) dans le séparateur (1), au-dessus du niveau de liquide (30) dans l'état d'équilibre.

17. Dispositif selon l'une des revendications 12 à 16 avec un capteur de mesure de pression (15).

18. Dispositif selon l'une des revendications 12 à 17 avec une conduite de gaz de lavage (19) pour évacuer le gaz du séparateur (1).

19. Dispositif selon la revendication 18, avec un capteur (14) dans la conduite de gaz de lavage (19) pour l'analyse de gaz.

20. Dispositif selon l'une des revendications 12 à 19, avec une seconde conduite d'amenée (32) pour l'amenée de gaz non dissous, qui relie le séparateur (1) au réservoir de liquide (48) et qui comporte dans une zone de conduite montante un capteur de niveau (3), et un dispositif de soupape (5) pour la sélection alternative de la première (36) ou de la seconde (32) conduite d'amenée.

21. Dispositif selon la revendication 20, avec lequel le dispositif de soupape comprend une soupape à trois voies (5) dont un raccordement est relié à un capillaire montant (2) menant au séparateur (1) et dont les deuxième et troisième raccordements sont reliés respectivement aux première (36) et seconde (32) conduites d'amenée.

22. Dispositif selon la revendication 20 ou 21, avec lequel la seconde conduite d'amenée (32) est apte à être reliée au dispositif de désaération (63) d'un relais collecteur de gaz (58) du réservoir de liquide de l'installation haute tension, et la première conduite d'amenée (36) est apte à être raccordée à la cuve (50) de l'installation haute tension.

23. Dispositif selon la revendication 20 ou 21, avec un tuyau collecteur de gaz (52) dont le dispositif d'aération (53) est relié à la seconde conduite d'amenée (32), le tuyau collecteur de gaz étant disposé juste au-dessus du réservoir de liquide dans une conduite montante (51, 54) qui relie la zone supérieure du réservoir de liquide (48) à la première conduite d'amenée (36).

24. Dispositif selon l'une des revendications 20 à 23, avec un collecteur de gaz (8) auquel mène une dérivation (38) de la seconde conduite d'amenée (2, 32), avec un raccordement (9) dans la zone supérieure en vue du prélèvement.
